# EUROPEAN PATENT APPLICATION

(11) **EP 2 497 493 A1**
(43) Date of publication of application: **12.09.2012**
(21) Application number: 10826901.0
(22) Date of filing: 01.11.2010
(51) Int. Cl.: A61K 39/04, A61K 35/76, A61K 39/00, A61K 48/00, A61P 31/06, C12N 15/09

(54) **NEBULIZABLE TUBERCULOSIS VACCINE FOR TRANSNASAL ADMINISTRATION COMPRISING PARAMYXOVIRUS VECTOR**

(30) Priority: 02.11.2009 JP 2009252218
(71) Applicant: Mie University, Mie 514-8507 (JP); Biocomo Co., Ltd., Mie-gun, Mie 510-1233 (JP); National Institute of Biomedical Innovation, Ibaraki-shi Osaka 567-0085 (JP)
(72) Inventor: YASUTOMI, Yasuhiro, Tsukuba-shi Ibaraki 305-0843 (JP); KAWANO, Mitsuo, Tsu-shi Mie 514-8507 (JP); NOSAKA, Tetsuya, Tsu-shi Mie 514-8507 (JP); FUKUMURA, Masayuki, Mie-gun Mie 510-1233 (JP)
(74) Representative: Paget, Hugh Charles Edward
(86) International application number: PCT/JP2010/069435
(87) International publication number: WO 2011/052771

(57) **Abstract**

Disclosed is a intranasal spray-type tuberculosis vaccine, which has a high prophylactic effect on human tuberculosis, particularly adult tuberculosis. The nebulizable tuberculosis vaccine for intranasal administration comprises a paramyxovirus gene (particularly rhPIV2) having, integrated therein, a gene encoding an α antigen derived from an acid-fast bacterium (e.g., an α antigen derived from Mycobacterium kansasii or Mycobacterium bovis BCG), an analogue of the gene, or a variant of the gene which has an equivalent function to that of the gene.

SEQUENCE LISTING

<110>  MIE university
       BIOCOMO CO., LTD.
       National Institute of Biomedical Innovation

<120>  Recombinant paramyxovirus

<130>  HP/FP6801963

<140>   EP10826901.0
<141>   01.11.2010

<150>   PCT/JP2010/069435
<151>   01.11.2010

<150>   JP 2009-252218
<151>   02.11.2009

<160>  12

<170>  PatentIn version 3.5

<210>  1
<211>  1396
<212>  DNA
<213>  Mycobacterium kansasii

<400>  1
gttaactatt ctttgtaccg ctccccgcct gccgccttct gccctgctcc gggtgcatag      60

cacccgtttg cgctccggat tatccgggcc gcaacggggc aacgggggaa gcggtggagt     120

ccgtcgccga ctcgcatagc accgttgctg tgttggcggg ggtaaccgat atcgaaatgg     180

aatgacttcg cgtcccgatc gacatttgcc ctactcacac ggtaagttct gccgggagca     240

cgcgagcaca tacggacaag gggcagggta tgacagacgt gagcgggaag attcgggcgt     300

ggggccgacg ccttctggtc ggcgcggccg ctgctgcggc ccttcctggc ctggtcggac     360

tcgccggcgg agcggcgacc gcgggagcgt tctcccgtcc cggcctgccg gtggagtacc     420

accaggtgcc gtcggctgcg atgggtcgca gtatcaaggt tcaattccaa agtggcggcg     480

acaactcgcc ggcggtgtac ctgctcgacg gtctccgcgc tcaagacgac tacaacggct     540

gggacatcaa caccccggcc ttcgagtggt actaccaatc gggcctgtcg gtcatcatgc     600

cggtcggcgg acagtccagt ttctacagtg actggtacag cccggcctgc ggcaaggccg     660

gctgcacgac ctacaagtgg gagaccttcc tgaccagcga gctgccgcaa tggctgtccg     720

cgaaccggag tgtcaagccc accggaagcg ccgcggtcgg catctcgatg gccggctcgt     780

cggccctgat cctgtccgtc taccacccgc agcagttcat ctacgcgggt tcgttgtcgg     840

ccctgatgga cccctcccag gggatggggc cgtctctgat cggcttggcg atgggtgacg     900

ccggtggtta caaggcctcg gacatgtggg gaccctcgag tgacccagcc tggcagcgta     960

acgacccgtc gctgcacatt ccggagctgg tcgccaacaa cacccgcctg tggatctact    1020

gcggcaacgg caccccgtcc gagttgggcg gtgccaatgt tccggccgaa ttcctggaga    1080

acttcgttcg cagcagcaac ctgaaattcc aggacgccta caacgccgcg ggcggccaca 1140

acgccgtgtt caatttggac gccaacggaa cgcacagctg ggagtactgg ggcgcgcagc 1200

tcaacgccat gaagggtgac ctgcaggcca gcctgggcgc ccgctgatcg cgcaacggtt 1260

gccgctactg ggcttgacgg caagacgccg tcaagccagt agtgtgttcg gcaccttgaa 1320

cgctggtccg ccatgttcaa cgagccggtc tacctgcccg caccgaacaa gctggtgaca 1380

tcgacccatg cggtgc 1396

<210> 2
<211> 285
<212> PRT
<213> Mycobacterium kansasii

<400> 2

Phe Ser Arg Pro Gly Leu Pro Val Glu Tyr His Gln Val Pro Ser Ala
1               5                   10                  15

Ala Met Gly Arg Ser Ile Lys Val Gln Phe Gln Ser Gly Gly Asp Asn
            20                  25                  30

Ser Pro Ala Val Tyr Leu Leu Asp Gly Leu Arg Ala Gln Asp Asp Tyr
        35                  40                  45

Asn Gly Trp Asp Ile Asn Thr Pro Ala Phe Glu Trp Tyr Tyr Gln Ser
    50                  55                  60

Gly Leu Ser Val Ile Met Pro Val Gly Gly Gln Ser Ser Phe Tyr Ser
65                  70                  75                  80

Asp Trp Tyr Ser Pro Ala Cys Gly Lys Ala Gly Cys Thr Thr Tyr Lys
                85                  90                  95

Trp Glu Thr Phe Leu Thr Ser Glu Leu Pro Gln Trp Leu Ser Ala Asn
            100                 105                 110

Arg Ser Val Lys Pro Thr Gly Ser Ala Ala Val Gly Ile Ser Met Ala
        115                 120                 125

Gly Ser Ser Ala Leu Ile Leu Ser Val Tyr His Pro Gln Gln Phe Ile
    130                 135                 140

Tyr Ala Gly Ser Leu Ser Ala Leu Met Asp Pro Ser Gln Gly Met Gly
145                 150                 155                 160

Pro Ser Leu Ile Gly Leu Ala Met Gly Asp Ala Gly Gly Tyr Lys Ala
                165                 170                 175

Ser Asp Met Trp Gly Pro Ser Ser Asp Pro Ala Trp Gln Arg Asn Asp

180                 185                 190

Pro Ser Leu His Ile Pro Glu Leu Val Ala Asn Asn Thr Arg Leu Trp
        195                 200                 205

Ile Tyr Cys Gly Asn Gly Thr Pro Ser Glu Leu Gly Gly Ala Asn Val
    210                 215                 220

Pro Ala Glu Phe Leu Glu Asn Phe Val Arg Ser Ser Asn Leu Lys Phe
225                 230                 235                 240

Gln Asp Ala Tyr Asn Ala Ala Gly Gly His Asn Ala Val Phe Asn Leu
                245                 250                 255

Asp Ala Asn Gly Thr His Ser Trp Glu Tyr Trp Gly Ala Gln Leu Asn
            260                 265                 270

Ala Met Lys Gly Asp Leu Gln Ala Ser Leu Gly Ala Arg
        275                 280                 285

<210>  3
<211>  978
<212>  DNA
<213>  Mycobacterium bovis BCG

<400>  3
atgacagacg tgagccgaaa gattcgagct tggggacgcc gattgatgat cggcacggca      60

gcggctgtag tccttccggg cctggtgggg cttgccggcg gagcggcaac cgcgggcgcg     120

ttctcccggc cggggctgcc ggtcgagtac ctgcaggtgc cgtcgccgtc gatgggccgc     180

gacatcaagg ttcagttcca gagcggtggg aacaactcac ctgcggttta tctgctcgac     240

ggcctgcgcg cccaagacga ctacaacggc tgggatatca acaccccggc gttcgagtgg     300

tactaccagt cgggactgtc gatagtcatg ccggtcggcg ggcagtccag cttctacagc     360

gactggtaca gcccggcctg cggtaaggct ggctgccaga cttacaagtg ggaaaccctc     420

ctgaccagcg agctgccgca atggttgtcc gccaacaggg ccgtgaagcc caccggcagc     480

gctgcaatcg gcttgtcgat ggccggctcg tcggcaatga tcttggccgc ctaccacccc     540

cagcagttca tctacgccgg ctcgctgtcg gccctgctgg acccctctca ggggatgggg     600

cctagcctga tcggcctcgc gatgggtgac gccggcggtt acaaggccgc agacatgtgg     660

ggtccctcga gtgacccggc atgggagcgc aacgacccta cgcagcagat ccccaagctg     720

gtcgcaaaca acacccggct atgggtttat tgcgggaacg gcaccccgaa cgagttgggc     780

ggtgccaaca tacccgccga gttcttggag aacttcgttc gtagcagcaa cctgaagttc     840

caggatgcgt acaacgccgc gggcgggcac aacgccgtgt tcaacttccc gcccaacggc     900

acgcacagct gggagtactg gggcgctcag ctcaacgcca tgaagggtga cctgcagagt     960

tcgttaggcg ccggctga                                                    978

<210>  4
<211>  325
<212>  PRT
<213>  Mycobacterium bovis BCG

<400>  4

Met Thr Asp Val Ser Arg Lys Ile Arg Ala Trp Gly Arg Arg Leu Met
1               5                   10                  15

Ile Gly Thr Ala Ala Ala Val Val Leu Pro Gly Leu Val Gly Leu Ala
            20                  25                  30

Gly Gly Ala Ala Thr Ala Gly Ala Phe Ser Arg Pro Gly Leu Pro Val
        35                  40                  45

Glu Tyr Leu Gln Val Pro Ser Pro Ser Met Gly Arg Asp Ile Lys Val
    50                  55                  60

Gln Phe Gln Ser Gly Gly Asn Asn Ser Pro Ala Val Tyr Leu Leu Asp
65                  70                  75                  80

Gly Leu Arg Ala Gln Asp Asp Tyr Asn Gly Trp Asp Ile Asn Thr Pro
                85                  90                  95

Ala Phe Glu Trp Tyr Tyr Gln Ser Gly Leu Ser Ile Val Met Pro Val
            100                 105                 110

Gly Gly Gln Ser Ser Phe Tyr Ser Asp Trp Tyr Ser Pro Ala Cys Gly
        115                 120                 125

Lys Ala Gly Cys Gln Thr Tyr Lys Trp Glu Thr Leu Leu Thr Ser Glu
    130                 135                 140

Leu Pro Gln Trp Leu Ser Ala Asn Arg Ala Val Lys Pro Thr Gly Ser
145                 150                 155                 160

Ala Ala Ile Gly Leu Ser Met Ala Gly Ser Ser Ala Met Ile Leu Ala
                165                 170                 175

Ala Tyr His Pro Gln Gln Phe Ile Tyr Ala Gly Ser Leu Ser Ala Leu
            180                 185                 190

Leu Asp Pro Ser Gln Gly Met Gly Pro Ser Leu Ile Gly Leu Ala Met
        195                 200                 205

Gly Asp Ala Gly Gly Tyr Lys Ala Ala Asp Met Trp Gly Pro Ser Ser
    210                 215                 220

Asp Pro Ala Trp Glu Arg Asn Asp Pro Thr Gln Gln Ile Pro Lys Leu
225                 230                 235                 240

Val Ala Asn Asn Thr Arg Leu Trp Val Tyr Cys Gly Asn Gly Thr Pro
                245                 250                 255

Asn Glu Leu Gly Gly Ala Asn Ile Pro Ala Glu Phe Leu Glu Asn Phe
            260                 265                 270

Val Arg Ser Ser Asn Leu Lys Phe Gln Asp Ala Tyr Asn Ala Ala Gly
        275                 280                 285

Gly His Asn Ala Val Phe Asn Phe Pro Pro Asn Gly Thr His Ser Trp
    290                 295                 300

Glu Tyr Trp Gly Ala Gln Leu Asn Ala Met Lys Gly Asp Leu Gln Ser
305                 310                 315                 320

Ser Leu Gly Ala Gly
                325

<210>  5
<211>  27
<212>  DNA
<213>  Artificial

<220>
<223>  PCR_P01

<400>  5
aagagctctt aaagggactt gatcttc                                          27

<210>  6
<211>  59
<212>  DNA
<213>  Artificial

<220>
<223>  PCR_P02

<400>  6
attttggcct gaacgttgat cttaacttct ttcttttttt tattaaattt atcatgtcc       59

<210>  7
<211>  49
<212>  DNA
<213>  Artificial

<220>
<223>  PCR_P03

<400>  7
aaaaaaagaa aagaagttaa gatcaacgtt caggccaaaa tattcagcc                  49

<210>  8
<211>  33
<212>  DNA
<213>  Artificial

<220>
<223>  PCR_P04

<400>  8
acttgatagg acggtaccca ttgagcctca atg                                    33


<210>  9
<211>  67
<212>  DNA
<213>  Artificial

<220>
<223>  PCR_P05

<400>  9
actgatcaat ctaacaaaaa aactaaacgt tctaagcacg aacccttaag gtgtcgtaac       60

gtctcgt                                                                 67


<210>  10
<211>  33
<212>  DNA
<213>  Artificial

<220>
<223>  PCR_P06

<400>  10
acttgatagg acggtaccca ttgagcctca atg                                    33


<210>  11
<211>  32
<212>  DNA
<213>  Artificial

<220>
<223>  PCR_P07

<400>  11
aaatttaaat atattcagcc atgcatcacc tg                                     32


<210>  12
<211>  36
<212>  DNA
<213>  Artificial

<220>
<223>  PCR_P08

<400>  12
aaatttaaat cttgtgatag atttcttaag atatcc                                 36

## Description

### TECHNICAL FIELD

The present invention relates to a intranasal spray-type tuberculosis vaccine, using a paramyxovirus, particularly a type 2 human parainfluenza virus vector (hPIV2).

### BACKGROUND ART

Tuberculosis is an infectious disease caused by Mycobacterium tuberculosis belonging to the genus Mycobacterium. Tuberculosis is currently one of the most common infectious diseases in the world. More than eight million people develop tuberculosis and about two million people die every year in the world. The reason for this is that the bacilli de Calmette et Guerin (BCG) vaccine which is a widely used tuberculosis vaccine is ineffective for adults and accordingly, an effect of tuberculosis prevention can be obtained only in younger generations by vaccination in infancy and childhood, and even implementation of additional vaccinations cannot prevent tuberculosis effectively in adults. More than 20,000 people develop tuberculosis every year in Japan (about 20 patients/one hundred thousands people), thus Japan is a middle-level tuberculosis prevalence country.

The BCG vaccine used for prevention of tuberculosis originates from Mycobacterium bovis. The Mycobactrium bovis loses toxity to human by successive culture for a long period of time, whereupon bacteria with only antigenicity are used as the BCG vaccine. When inoculated with the live attenuated bacteria, humans can acquire immunity preventing infection of Mycobacterium tuberculosis. The BCG vaccine is the only vaccine for tuberculosis prevention that is widely accepted as having an efficacy for prevention of infantile tuberculosis.
The efficacy of BCG vaccine lasts for dozen years or so, and prevention of adult tuberculosis relies upon an increase in natural immunity. However, natural immunity cannot suppress the onset of adult tuberculosis. It is known that adulthood BCG vaccination has a lower or almost no efficacy for adult tuberculosis.

### PRIOR ART DOCUMENTS

### PATENT DOCUMENTS

Patent Document 1: International Publication WO 2002/06655
Patent Document 2: Japanese Patent Application Publication JP-A-2008-074749

### NON-PATENT DOCUMENTS

Non-Patent Document 1: 52nd Annual Meeting of the Japanese Society for Virology, "M Protein Deficient Parainfluenza type 2 (PIV2) and Characterization of the PIV2 harboring mouse Interleukin-4," 21st November 2004

### SUMMARY OF THE INVENTION

### PROBLEM TO BE OVERCOME BY THE INVENTION

In consideration of the aforementioned state of affairs, the development of several adult tuberculosis vaccines has been advanced. For example, under development are MVA85A vaccine (vaccine expressing 85A antigen of acid-fast bacterium on vaccinia virus Ankara), Aeras-402 vaccine (vaccine expressing 85A antigen and TB10.4 on type 35 adenovirus) and M72F (otherwise known as GSK TB101: a vaccine developed by GSK (Glaxo Smith Kline plc) and using a protein of Mycobacterium tuberculosis with GSKAS02 Adjuvant) and the like. However, no adult tuberculosis vaccine has been hitherto known that has a sufficient efficacy.
The present invention was made in view of the foregoing circumstances and an object thereof is to provide a intranasal spray-type tuberculosis vaccine, which has efficacy in prevention of tuberculosis.

### MEANS FOR OVERCOMING THE PROBLEM

HPIV2 is a virus belonging to Family Paramyxoviridae and has genome that is a single negative-stranded RNA having about 15000 bases. The nucleocapsid protein (NP) is combined with this nucleic acid to be formed into a helically symmetric ribonucleoside protein complex (ribonucleocapsid, RNP). M protein, one of proteins encoded by the virus, interacts with an intracytoplasmic domain of a viral glycoprotein, lipid bilayer envelope and RNP, and is important for budding of viral particle. Recombinant virus of which M protein gene has been deleted by gene manipulation can infect cells to express host viral proteins but cannot bud. The present inventors found that M protein-deficient PIV2 could be used as a vector to express exogenous protein in a target cell (non-patent document 1).

Meanwhile, the inventors found that an α antigen derived from acid-fast bacteria had efficacy in prevention and treatment of allergic diseases (Patent Document 1). The α antigen expression vector was constructed by using pcDNA-α-K having a CMV promoter and a TPA signal peptide, and the effect in asthmatic mouse models was investigated. The inventors further found that PIV2 vector expressing α antigen has efficacy against allergic diseases, since PIV2 vector has high ability of α antigen induction (Patent Document 2).
The inventors examined whether or not the above-described inventors' technique was applicable to a tuberculosis vaccine. The inventors found that the expression vector made by integrating the α antigen into hPIV2 (rhPIV2) deficient in M protein or F protein exhibited efficacy for prevention of tuberculosis, regarding infection prevention of tubercle bacillus against which conventional methods had almost no efficacy, whereupon the inventors basically completed the present invention.

Thus, a intranasal spray-type tuberculosis vaccine according to a first invention comprises a gene encoding an α antigen derived from Mycobacteria (Ag85B), an analog thereof or a mutant thereof having a function similar thereto (hereinafter called "α antigen or the like"), said gene being integrated into a paramyxovirus gene deficient in an M gene, F gene or HN gene.
Furthermore, a method of prevention or treatment of tuberculosis, according to a second invention, comprises integrating a gene encoding an α antigen derived from Mycobacteria, an analog thereof or a mutant having a function similar thereto into a paramyxovirus gene deficient in an M gene, F gene or HN gene, and nebulizing a human or mammals for intranasal administration.
The paramyxovirus represents virus belonging to Family Paramyxoviridae. The paramyxovirus represents a class of virus that has minus stranded RNA as a genome and virions ranging from 150 nm to 350 nm and presents a multiform appearance. The paramyxovirus virions are enveloped and have two types of glycoproteins (F and HN) arranged on the surface of the envelope. The paramyxovirus includes parainfluenza virus (PIV), bovine parainfluenza virus-3, Sendai virus, mumps virus, canine distemper virus, rinderpest virus, morbillivirus, peste-des-petits-ruminants virus, Newcastle disease virus, Avian paramyxovirus type 2-9, Hendra virus, Nipah virus, bovine respiratory syncytial virus, human respiratory syncytial virus, human metapneumovirus, and the like.
It is preferred that the α antigen is derived from Mycobacterium kansasii or Mycobacterium bovis BCG. Furthermore, it is preferred that the analog of the α antigen is an 85 complex-forming protein 85A or 85 complex-forming protein 85C.

### EFFECT OF THE INVENTION

According to the present invention, a vaccine having a high efficacy in prevention and treatment of adult tuberculosis can be provided.
The vaccine according to the invention is a intranasal spray-type tuberculosis vaccine and has a high administration easiness while utilizing a high protein expression signature of paramyxovirus central to the virus (particularly, hPIV2) in respiratory infections. Although recombinant vaccines of MVA85A and Aeras402 currently under development are injection products, the intranasal spray-type tuberculosis vaccine according to the invention is an aerosolized agent and has a higher expression level of target protein than injection solution administration. Induction of local immunity in the lung as a disease expression site is more important than systemic immunization in tuberculosis prevention. A high cell-mediated immunity induction in the lung is obtained by the use of a paramyxovirus vector into which a protein gene having a cell-mediated immunity (Th1) skewing function is inserted, whereby it can be considered that a higher vaccine efficacy against tuberculosis bacteria as compared with other vectors. Furthermore, the vector based on the vaccine has a high safety particularly for adults in virus pathogenicity.
Furthermore, a high yield and reduction in costs can be realized by the use of cultivated cell and nebulization in the production aspect.

### BRIEF DESCRIPTION OF THE DRAWINGS

[FIG. 1] A view explaining outline of antisense rhPIV2 genome that does not express M protein by insertion of stop codon;
[FIG. 2] A view explaining a method of recovering virus particles from the plasmid vector of M gene-deficient type antisense rhPIV2 genome cDNA by insertion of stop codons, driven by a T7 promoter;
[FIG. 3] A view showing the structure of ΔM/rhPIV2 and ΔF/rhPIV2 which is deficient in M protein gene or F protein gene and into which gene such as Ag85B or the like is inserted as an expression type;
[FIG. 4] A photomicrograph which confirmed expression of GFP in a respiratory tract of hamster to which rhPIV2-GFP has been administered intranasally;
[FIG. 5] A photograph of western blotting showing expression of the gene product after rhPIV2-Ag85B transduction;
[FIG. 6] A view showing a state where ΔF/rhPIV2 with GFP gene grows in the cells expressing F protein, is revealed by increased GFP (+) cells;
[FIG. 7] A view of western blot showing BCG-Ag85B being expressed by ΔF/rhPIV2 vector;
[FIG. 8] Photographs demonstrating nodes of tubercle bacilli in the lung of the mice after vaccination with rhPIV2-Ag85B (M-stop), followed by the challenge with tubercle bacilli. (A) shows the case where control (rhPIV2-GFP) was administered four times. (B) shows the case where expression type Ag85B-DNA was administered twice and rhPIV2-Ag85B (M-stop) was administered twice. (C) shows the case where rhPIV2-Ag85B (M-stop) was administered four times;
[FIG. 9] Photomicrographs showing lesion of the lung of FIG. 8. (A) shows the mouse lung infected with tubercle bacilli after rhPIV2-Ag85B (M-stop) had been administered four times. (B) shows the mouse lung infected with tubercle bacilli after control (rhPIV2-GFP) had been administered four times; and
[FIG. 10] A graph showing comparison of the number of tubercle bacilli in a tuberculosis infection prevention test with rhPIV2-Ag85B (M-stop).

### BEST MODE FOR CARRYING OUT THE INVENTION

Embodiments according to the present invention will be described with reference to the drawings. The technical scope of the invention should not be limited by the embodiments, which can be practiced in various forms without changes in the scope of the invention. The technical scope of the invention covers the range of equivalence.
Human paramyxovirus (particularly, hPIV2) used in the present invention has an exceedingly low pathogenicity and a high protein expression. Accordingly, it is possible to prepare and use a paramyxovirus gene into which a gene encoding α antigen or the like as foreign protein is integrated.
However, the hPIV2 vector becomes a safer hPIV2 vector that does not produce infectious hPIV2 by use of rhPIV2 deficient in an M gene, F gene or HM gene although infection and expression of protein can equivalently be carried out, following an adenovirus vector from which composite protein genes of viruses have been deleted and to which the genes have been supplied from cells, plasmid or the like thereby to supply a safer nongrowing virus vector. Thus, genes encoding α antigen are integrated into hPIV2 having deletion of the M gene, F gene or HM gene in the present invention. The α antigen or the like may be inserted into any location of rhPIV2 genome. Since an expression level of foreign protein is increased, it is preferable to insert the α antigen or the like into 5' distal end side of an antisense virus genome. The antisense virus genome is a single-stranded RNA and has a structure of leader (promoter sequence), NP, V/P, M, F, HN, L and trailer from the 5' end to 3' end. Accordingly, it is preferable that the gene encoding α antigen or the like is located shortly in the rear of the leader (promoter sequence) or in the vicinity of 5'end side between NP V/P or the like.

In order that M gene that is a matrix protein and F gene composing a membrane protein or HN gene may be knocked out, exemplified are a method of removing all or part of gene encoding M protein, F protein or HN protein, a method of inserting a stop codon into any location of gene encoding M protein, F protein or the HN protein, and the like. The replication-deficient PIV2 which does not carry out multiple-stage growth in cells and tissues which do not express the genes can be prepared by a method of transiently introducing plasmid expressing M protein, F protein or HN protein thereby to express the gene or by developing cells expressing M gene, F gene or HN gene thereby to proliferate PIV2 which is deficient in M gene, F gene or HN gene on the cells. Viruses that are completely absent of M gene, F gene or HN gene such that the viruses cannot autonomically replicate are called ΔM/rhPIV2 or rhPIV2(ΔM), ΔF/rhPIV2 or rhPIV2 (ΔF) or ΔHN/rhPIV2 or rhPIV2 (ΔHN) respectively. In the present invention, an α antigen (Ag85B) is one of proteins that are common in acid-fast bacilli and is identified as antigen 85 complex constituting protein 85B that is one of antigen 85 complexes. The antigen 85 complex includes antigen 85 complex constituting protein 85A having molecular weight of about 30 to 32 kD (Ag85A: Infect. Immun. 57: 3123-3130, 1989), antigen 85 complex constituting protein 85B having molecular weight of about 30 to 32 kD (Infect. Immun. 58(2). 550-556 (1990). Accession No. X53897), and antigen 85 complex constituting protein 85C (Ag85C: Infect. Immun. 59: 5205-3212, 1991). These proteins are major secreted proteins of acid-fast bacilli. These secreted proteins are known to have a gene-amino acid sequence with a high homology transcending species and cross reactivity with monoclonal antibody (Microbiol. Rev. 56: 648-661, 1992). An analog of the α antigen (antigen 85 complex constituting protein 85B: Ag85B) includes antigen 85 complex constituting protein 85A (Ag85A), antigen 85 complex constituting protein 85C (Ag85C).

In the present invention, the gene encoding α antigen derived from acid-fast bacilli or the analog thereof indicates a gene capable of expressing α antigen protein or analog thereof such as the antigen 85 complex constituting protein 85A or antigen 85 complex constituting protein 85C. More specifically, a gene is exemplified that is in the form of an expression vector and includes a gene encoding an α antigen or analog thereof. As a gene encoding the α antigen are exemplified Mycobacterium kansasii (Infect. Immun. 58: 550-556, 1990), Mycobacterium bovis BCG (J. Bacteriol. 170:3847-3854, 1988), Mycobacterium avium (Infect. Immun. 61: 1173-1179, 1993), Mycobacterium intracellulare (Biochem. Biophys. Res. Commun. 196: 1466-1473, 1993), Mycobacterium leprae (Mol. Microbiol. 6: 153-163. 1992) and the like. All of these genes can be used in the present invention. Of these, as genes encoding an α antigen of Mycobacterium kansasii is exemplified a DNA having the base sequence from positions 390 to 1244 of SEQ ID No: 1 and as genes encoding an α antigen of Mycobacterium bovis BCG is exemplified a DNA having the base sequence from positions 121 to 975 of SEQ ID No: 3. Other DNAs include a mutant DNA that hybridies to a DNA with a sequence complementary to this DNA under a stringent condition and a mutant DNA comprising a DNA encoding a protein having an amino acid sequence in which one or more than one (preferably, several) amino acids have been substituted, deleted and/or added to the amino acid sequence of the protein encoded by this DNA, wherein the mutant encodes a protein having the same function as the Mycobacterium kansasii-derived or Mycobacterium bovis BCG-derived α antigen. The aforementioned same function as the Mycobacterium kansasii-derived or Mycobacterium bovis BCG-derived α antigen indicates achieving the same preventive efficacy against the infection of Mycobacterium kansasii.
The α antigen of Mycobacterium bovis BCG comprises a mature protein of 285 amino acids, and this amino-acid sequence has a higher amino-acid homology (100%) than the α antigen of Mycobacterium kansasii due to Mycobacterium tuberculosis. It is considered that a higher protective efficacy of tuberculosis incidence will be achieved by introduction of the α antigen.

A specific method of obtaining a mutant DNA includes the following one. Thus, a colony hybridization in the presence of 50% formamide, 4×Denhardt, 5×SSPE (SSPE solution: EDTA sodium phosphate (SSPE), 1×Denhardt: 0.02% Ficoll, 0.02% polyvinyl pyrrolidone, 0.02% bovine serum albumin), 0.2% SDS, 100 µg/mL ssDNA, and 12.5 ng of a probe (12.5 ng of a purified cDNA fragment having a base sequence from positions 390 to 1244 of SEQ ID No: 1 or a base sequence from positions 121 to 975 of SEQ ID No: 3 labeled with [α-³²P]dCTP (Amersham) using the BcaBest DNA labeling kit (Takara) at 45°C for 14-16 hours, the filter is washed in 1×SSPE and 0.5% SDS solution at 45°C/30 min, then in 0.1×SSPE and 0.5% SDS solution at 65°C/1 hour to eliminate the background completely, which is then exposed to an X-ray film (Fuji) at -80°C for 72 hours to detect the positions of the corresponding colonies and to isolate the single colony, thus the mutant DNA can be obtained. These mutants are those for which amino acid sequences encoded thereby normally have a homology of 60% or greater with the amino acid sequence of the α antigen protein, and preferably a homology of 75% or greater. In the case of genes encoding the α antigen other than Mycobacterium kansasii or Mycobacterium bovis BCG, they may be mutants thereof as well.

As genes encoding the antigen 85 complex-forming protein 85A which is an analog of the α antigen, there can be mentioned genes derived from the Mycobacteria similar to various Mycobacteria described above for the α antigen gene. More specifically, there can be mentioned a DNA encoding the antigen 85 complex-forming protein 85A derived from Mycobacterium tuberculosis (Infect. Immun. 57: 3212-3130, 1989). For genes encoding the antigen 85 complex-forming protein 85C as well, there can be mentioned a gene derived from various Mycobacteria, and more specifically, there can be mentioned a DNA encoding the antigen 85 complex-forming protein 85C derived from Mycobacterium tuberculosis (Infect. Immun. 59: 3205-3212, 1991). For these DNAs as well, as described antibody, they may be a mutant DNA that hybridizes to this DNA under a stringent condition, or a mutant DNA comprising a DNA encoding a protein having an amino acid sequence in which one or more than one (preferably, several) amino acid residues have been substituted, deleted and/or added to the amino acid sequence of the protein encoded by this DNA, wherein said mutant encodes a protein having the same function as the antigen 85 complex-forming protein 85A or the antigen 85 complex-forming protein 85C.

The above DNAs can be cloned by a method such as RT-PCR reaction on mRNA derived from Mycobacteria using as PCR primers appropriate DNA segments based on the sequence information of Genebank, etc. Chemical synthesis is also possible based on the amino acid sequence information. Furthermore, the above DNA mutants may easily be obtained using, for example, site-directed mutagenesis, PCR method, a common hybridization method, or the like.

In the present invention, the α antigen protein derived from Mycobacteria or derivatives thereof such as antigen 85 complex-forming protein 85A or antigen 85 complex-forming protein 85C or mutant proteins thereof can be used for the prevention of tuberculosis. As such α antigens, there can be mentioned proteins encoded by genes encoding the above-mentioned α antigen. More specifically, there can be mentioned an α antigen which is encoded by the DNA having the base sequence derived from Mycobacterium kansasii described in SEQ ID NO: 1 and which has the amino acid sequence of SEQ ID NO: 2 or an α antigen which is encoded by the DNA having the base sequence derived from Mycobaterium bovis BCG described in SEQ ID NO: 3 and which has the amino acid sequence of SEQ ID NO: 4. The latter base sequence and amino acid sequence can be obtained by visiting this URL (http://www.ncbi.nlm.nih.gov/nuccore/X62397?ordinalpos=1&i tool=EntrezSystem2.PEntrez.Sequence.Sequence_ResultsPanel.Sequ ence_RVDocSum).
In addition to the α antigen having this amino acid sequence, a mutant protein comprising an amino acid sequence in which one or more than one (preferably, several) amino acid residues have been substituted, deleted and/or added to the amino acid sequence of SEQ ID NO: 2 or SEQ ID NO: 4, said protein having the same function as the α antigen may be used. In the case of genes encoding the α antigen other than Mycobacterium kansasii or Mycobacterium bovis BCG, a mutant protein comprising an amino acid sequence in which one or more than one (preferably, several) amino acid residues have been substituted, deleted and/or added to the amino acid sequence and which has the same function as the α antigen as well can be used. Furthermore, for the antigen 85 complex-forming protein 85A or the antigen 85 complex-forming protein 85C as well, the antigen 85 complex-forming protein 85A derived from Mycobacterium tuberculosis (Infect. Immun. 57: 3123-3130, 1989), the antigen 85 complex-forming protein 85C derived from Mycobacterium tuberculosis (Infect. Immun. 59: 3205-3212, 1991) and the like can be used. Other the analogs of these α antigen proteins, mutants similar to those of the α antigen protein mentioned above can be used.

These proteins may be produced by a recombinant DNA technology using genes encoding them or by chemical synthesis. Alternatively, Mycobacteria such as Mycobacterium kansasii, Mycobacterium bovis BCG may be cultured in a suitable medium, and the proteins may be purified from the culture medium by a known purification method (Scand. J. Immunol. 43: 202-209, 1996; J. Bacteriol. 170: 3847-3854, 1988; Hiroshima J. Med. Sci. 32: 1-8, 1983; J. Bacteriol. 170: 3847-3854, 1988).
In the present invention, when the gene encoding Mycobacterium-derived α antigen, analogs thereof or mutants thereof are used as tuberculosis vaccine, they are used in the form of hPIV2 containing the gene encoding Mycobacterium-derived α antigen, analogs thereof or mutants thereof. An hPIV2 genome deficient in M, F or HN gene can be used as the hPIV2. An rhPIV2 vector expressing α antigen etc. is constructed by integrating a gene encoding the α antigen etc. into the M, F or HN gene-deleted hPIV2 (rhPIV2) genome.

This expression vector can generally be administered as an aerosolized agent to mammals including human. The aerosolized agent can be prepared by conventional methods. For example, after having been dissolved into a suitable solvent (a buffer solution such as PBS, normal saline, stabilizing agent or the like), the expression vector is filter-sterilized, if necessary and is then caused to fill an aseptic container, thereby being prepared as the aerosolized agent. A commonly used carrier or the like may be added to the aerosolized agent, if necessary. In order to use the expression vectors thus obtained as the tuberculosis vaccine, the aerosolized agent can be administered under normal use of the aerosolized agent.

A dose of tuberculosis vaccine can vary depending upon patient's weight or the like. As a vaccine, about 1×10³ to 1×10¹⁰ viruses, preferably about 1×10⁴ to 1×10⁹ viruses are normally used as the expression vector per dose. The expression vector is preferably administered twice to five times at intervals of several days to several weeks.
The present invention will be described concretely by way of examples as follows. However, the present invention should not be limited to the examples.

### EXAMPLE 1

### Construction of M protein-deleted antisense rhPIV2 (M-stop) by introduction of stop codon

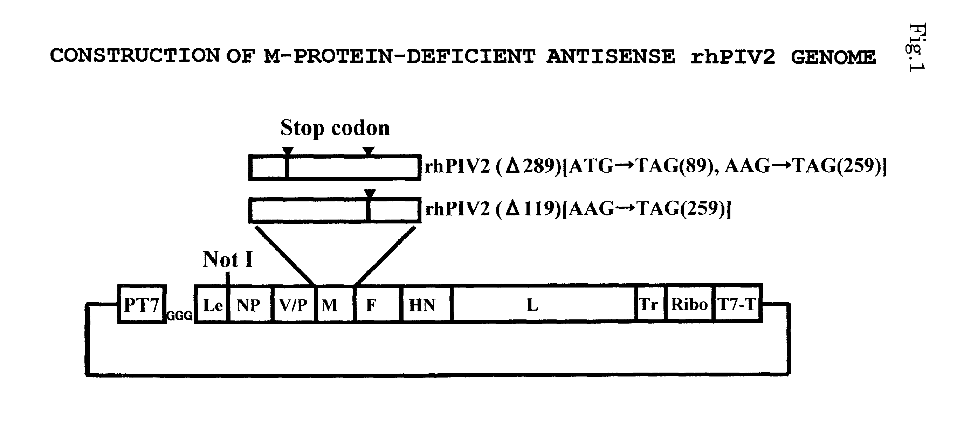

FIG. 1 shows an outline of antisense rhPIV2 (M-stop) genome in which a stop codon is introduced into a predetermined location of the gene encoding M protein. A plasmid vector is shown in which two types of M protein deletion hPIV2 (rhPIV2 (Δ119) and rhPIV2 (Δ289)) having Δ119 (the 259th AAG of M protein gene has been changed to TAG) and Δ289 (the 89th ATG of M protein gene has been changed to TAG and the 259th AAG has been changed to TAG) respectively. Genetic techniques known to skilled persons (PCR method, for example) can be used to construct hPIV2 (M-stop) deficient in M protein.

### EXAMPLE 2

### Method of preparing rhPIV2 (M-stop)

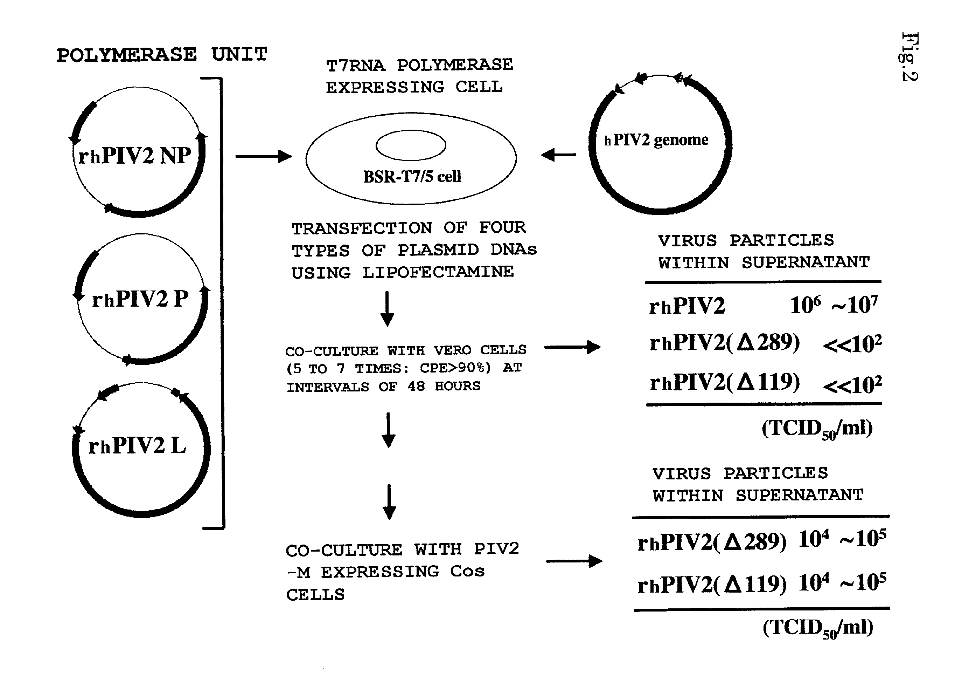

Next, the following will describe a method of recovering virus particles from the plasmid including the antisense rhPIV2 genome cDNA inserted downstream of T7 promoter. FIG. 2 shows the outline of the recovery method. The antisense rhPIV2 genomes (rhPIV2, rhPIV2 (Δ289) and rhPIV2 (Δ119) were transfected into cells (for example, BSR-T7/5) expressing T7 RNA polymerase. In this case, three types of expression vectors hPIV2-NP, hPIV2-P and hPIV2-L expressing each hPIV2 polymerase unit (that is, NP protein, P protein and L protein) were co-transfected. Methods known to skilled persons can be used for transfection of DNA (Lipofectamine was used in the embodiment).

When the transfected cells were co-cultured with Vero cells 5 to 7 times at intervals of 48 hours, a cytopathic effect (CPE) was confirmed with 90% efficiency. In this case, a large amount of recombinant virus particles were obtained in use of hPIV2. However, virtually no recombinant virus particles were detected in use of rhPIV2 (Δ289) and rhPIV2 (Δ119). This shows that hPIV2 did not bud when M protein was deficient.
Instead of Vero cells, co-cultivation was then carried out by the use of Cos cells expressing hPIV2-M (Cos cells to which hPIV2-M that was a vector expressing M protein was transfected). As a result, a large amount of recombinant virus particles were detected in use of rhPIV2 (Δ289) and rhPIV2 (Δ119).
As the method of preparing rhPIV2 using the T7 promoter has been described above, rhPIV2 prepared using any vector (phage vector, plasmid vector, etc.) may be used.

### EXAMPLE 3

### Construction of antisense genome (ΔM/rhPIV2, ΔF/rhPIV2) completely deficient in M or F gene

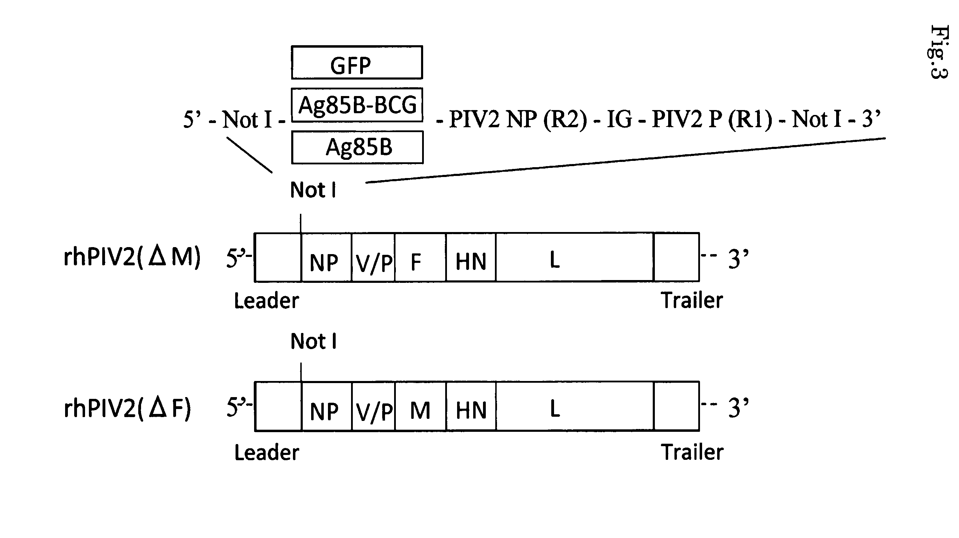

As shown in FIG. 3, about 5.5 kb gene between Sacl-Kpnl restriction enzyme sites encoding parts of M, F and HN genes of plasmid including hPIV2 genome genes was introduced into Sacl-Kpnl restriction enzyme site of pUC118 vector.

Regarding construction of M gene deletion genome, ΔM/rhPIV2 which was completely deficient in M gene coding region including R1 and R2 regions of M gene was prepared. Primers were synthesized which connected M-gene-deficient upstream and downstream sequences. Multiple PCRs were carried out to construct ΔM/rhPIV2 from which the M gene coding region had been deleted completely. The primer sequence included 5'-tgaaggagat cattgactc ttaaagggac ttg -3' (sequence No. 5) as a sequence including the restriction enzyme site, 5'-tggaacgttt agttttttta ttaaatttat catgtccagc ct-3' (sequence No. 6) as an M gene upstream region sequence, 5'-ttaataaaaa aactaaacgt tccacaataa atcaacgttc ag-3' (sequence No. 7) as an M gene downstream region sequence and 5'-ggattcttaa agcttggatg gaga-3' (sequence No. 8). The total number of bases was adjusted so as to be multiples of 6.

Regarding construction of F gene deletion genome, ΔF/rhPIV2 was made from which an F gene coding region 1,884 bp including R1 and R2 regions completely deficient in F gene. Regarding the F gene-deleted genome, Bcll restriction enzyme sequence is located in F structural gene region and Kpn1 restriction enzyme sequence is located in the HN gene. Complete deficiency of the F gene was carried out using the restriction enzyme sequence. Primers were constructed using sequences of 5'-actgatcaat ctaacaaaaa aactaaacgt tctaagcacg aacccttaag gtgtcgtaac gtctcgt-3' (sequence No. 9) and 5'-acttgatagg acggtaccca ttgagcctca atg-3' (sequence No. 10).

### EXAMPLE 4

### Recovery of transient virus

Regarding the system of transiently expressing and recovering ΔM/rhPIV2 or ΔF/rhPIV2 virus into which α antigen gene for tuberculosis vaccine was introduced and which was completely deficient in M or F gene, the virus was recovered by a method similar to the rhPIV2 (M-stop) virus recovery method or by another method.

### EXAMPLE 5

### Construction of M or F protein expressing cells

When M protein or F protein of hPIV2 is constantly expressed in cells, it is considered that the protein has a cell toxity. Accordingly, we constructed (1) the system which constantly expressed the protein, and (2) the system which inductively expressed the protein.

### (1) Construction of protein constantly expressing system

The construction was carried out using a vector which held a neomycin-resistant gene or a puromycin-resistant gene and into which an M or F gene sequence was introduced into the vector holding chicken β-actin promoter sequence and rabbit β-globin poly A sequence (CAG promoter).

### (2) Construction of protein inducible expression system Construction of M or F protein expression cell by inducible expression system

The construction was carried out by removing stuffer sequences located in two loxP sequences respectively by an inducible expression system Cre recombinase, using an expression inducing system (pCALN vector). A foreign gene was insertable into an Swal restriction enzyme site of this vector. The blunt end by restriction enzyme Swal reaction was used to construct an expression vector. In the case of M gene, primers for PCR in which the PCR products by using the primers set contained M gene and Pme1 enzyme sites both 5' and 3' ends to make blunt ends by restriction enzyme Pme1 were used, whereby the M gene inducible expression vector was constructed.

Regarding an F gene inducible expression system, primers for PCR in which the PCR products by using the primers set contained F gene and Swa1 enzyme sites both 5' and 3' ends to make blunt ends by restriction enzyme Swa1 were used, whereby the F gene inducible expression vector was constructed. PCR primer sequences for the F gene recovery were 5'-aaatttaaat atattcagcc atgcatcacc tg-3' (sequence No. 11) and 5'-aaatttaaat cttgtgatag atttcttaag atatcc-3' (sequence No. 12).

### EXAMPLE 6

### Construction of expression cells

The vectors were trasnfected into Vero cells using Lipofectamine 2000 (invitrogen) or the like and the cells were cultured with neomycin (1 mg/ml). Screening was carried out by the drug resistance against neomycin. Vero cells which were capable of inducible M gene expression were recovered. The cells were infected with an adenoviruses expressing Cre protein so that M proteins were expressed. The cells were infected with the M gene-deleted virus recovered in the previous embodiment so that non-proliferated viruses were recovered. Consequently, M-gene-deficient non-proliferated viruses were recovered.

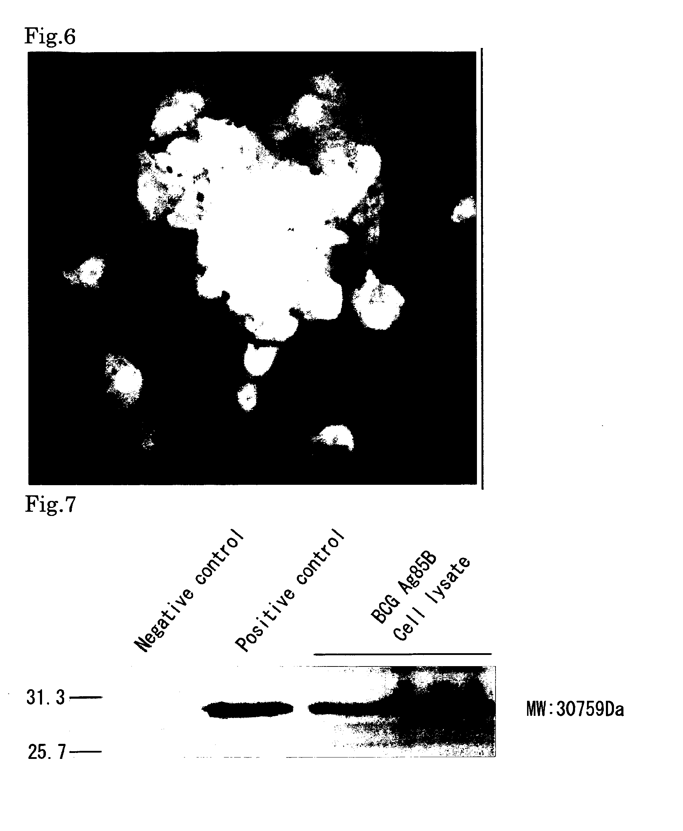

F protein inducible expression cells or F protein regular expression cells were established in a manner similar to M protein expression cells or in another manner. Consequently, F protein inducible expression cells and F protein regular expression cells were obtained. F gene deleted replication-deficient viruses were recovered (FIG. 6).

### EXAMPLE 7

### Construction of rhPIV2 including genes encoding α antigen

A signal sequence of TPA was added to 5' of α antigen genes (αK) of Mycobacterium kansasii comprising the sequence from positions 390 to 1244 of SEQ ID NO: 1, and R1 of hPIV2, an intervening sequence and R2 were added to 3'and inserted into a Not I site of rhPIV2 so that rhPIV2 into which α antigen genes were integrated (hereinafter, "rhPIV2-Ag85B" or "rhPIV2/Ag85B") was constructed (see FIG. 3). The Not I site was integrated just after a leader sequence of rhPIV2 genome and provided in the 5' end of the antisense rhPIV2 genome. Additionally as a control gene, rhPIV2 where GFP (green fluorescent protein derived from Aequorea victoria) gene was inserted into the Not I site was constructed (hereinafter, "rhPIV2-GFP" or "rhPIV2/GFP").

A large amount of rhPIV2-Ag85B and rhPIV2-GFP were prepared by the method of embodiment 2.

Subsequently, regarding genes of α antigen gene (αK) BCG-Ag85B of Mycobacterium bovis BCG comprising the sequence from positions 121 to 975 of SEQ ID NO: 3, DNA having the sequence from positions 121 to 975 of SEQ ID NO: 3 was added to a start codon (ATG) and inserted into the Not I site of rhPIV2 so that rhPIV2 into which BCG-Ag85B was integrated (hereinafter, "ΔM/rhPIV2-Ag85B (BCG)" or "ΔF/rhPIV2-Ag85B (BCG)") was constructed (see FIG. 3). The Not I site was integrated just after the leader sequence of rhPIV2 genome and provided in the 5'end of antisense rhPIV2 genome.

More specifically, regarding Ag85B of BCG, a signal sequence region (1 to 40) in an amino acid sequence of sequence No. 4 was deleted and the sequence from positions 121 to 975 (ttctccc···) which encodes the amino acids from positions 41 to 325 (FSR ...) was integrated.

### Example 8

### Confirmation of expression of GFP in hamsters

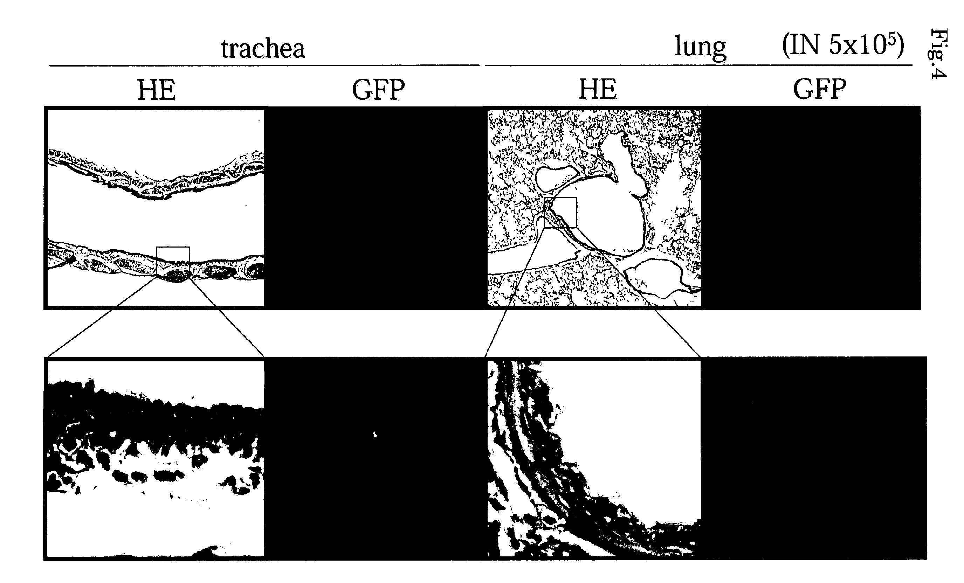

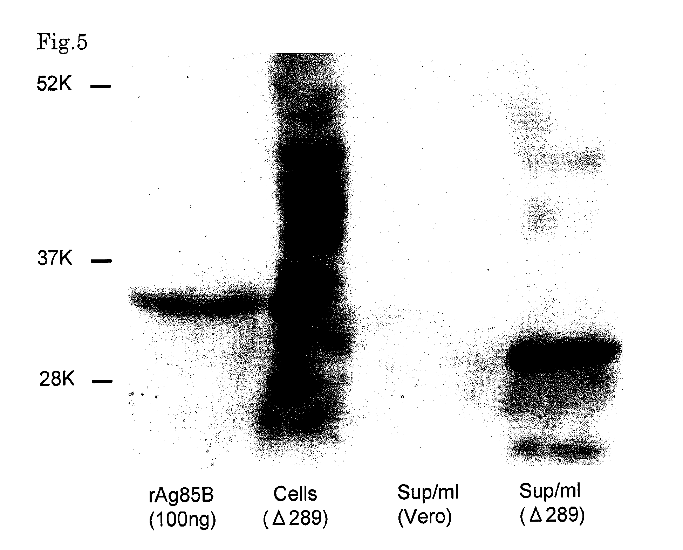

When rhPIV2-GFP was intranasally administered to hamsters (IN: 5×10⁵ viruses), fluorescence of GFP was detected in epithelial cells of trachea and lung as shown in FIG. 5. This revealed that rhPIV2 showed exceedingly high level expression of foreign genes in trachea and lung by the intranasal administration (FIG. 4).

### EXAMPLE 9

### Confirmation of efficacy about prevention of tuberculosis using mice

30 BALB/C mice were divided into three groups A, B and C with 10 mice per group, and the following treatments were conducted on the respective groups:

### Group A (control group)

The rhPIV2-GFP was intranasally administered four times at the intervals of two weeks. An applied dose of rhPIV2-GFP by intranasal administration was 1×10⁷ viruses/20 µL per administration.

### Group B (Test group 1)

An expression-type Ag85B-DNA was intranasally administered to an abdominal cavity twice at the intervals of two weeks, and rhPIV2-Ag85B (M-stop) was intranasally administered twice at the intervals of two weeks. An expression-type Ag85B-DNA by intranasal administration was 50 µg per mouse. Furthermore, an applied dose of rhPIV2-Ag85B (M-stop) by intranasal administration was 1×10⁷ viruses/20 µL per administration.

### Group C (Test group 2)

The rhPIV2-Ag85B (M-stop) was intranasally administered four times at the intervals of two weeks, and an applied dose of rhPIV2-Ag85B (M-stop) by intranasal administration was 1×10⁷ viruses/20 µL per administration.

In each group, highly-virulent tubercle bacillus (Mycobaterium tuberculosis Kurono strain) was intranasally infected one week after the last immunity. Lungs and spleens were removed 49 days after the start of experiment so that tubercle, a microscopic appearance of lesion cutout and the number of tubercle bacilli were confirmed.

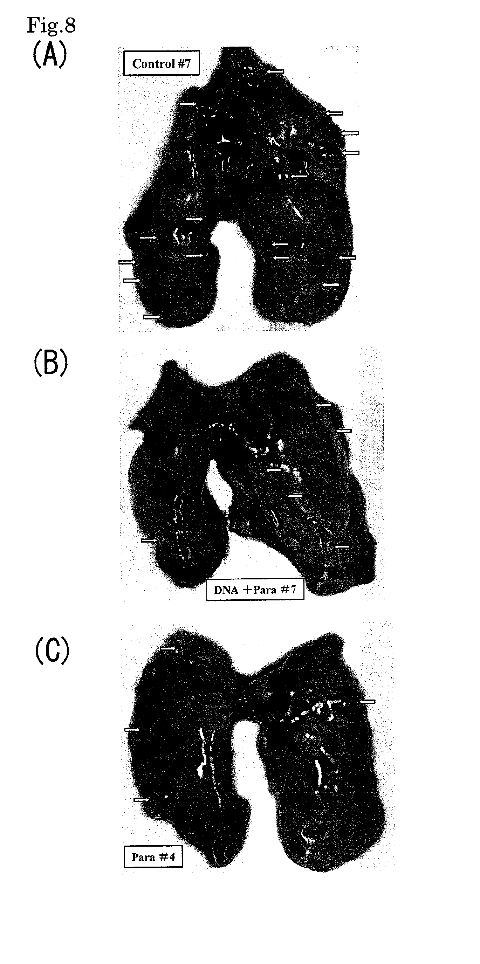

The results were shown in FIGS. 8 to 10. The greatest characteristic of an intranasal nebulization type vaccine is that inoculation is easier and has a higher safety as compared with vaccination by injection or the like. The tuberculosis vaccine using rhPIV2 can be administered by the use of currently available throat spray type, whereupon a speedy and safe inoculation can be carried out. In particular, when shortage of physicians, infection through injection and the like are taken into consideration, the intranasal nebulization is thought to have a profound effect in vaccination in developing countries etc. and to have a high world-wide impact.

The tubercle bacillus is one of intracellular eubacteria that grow in phagocytes such as macrophage. Cell-mediated immunity (Th1) that induces activated macrophage and cytotoxic T cell (CTL) becomes central to the prevention of infection of tubercle bacillus but not humoral immunity which consists mainly of antibody. Ag85B is a single protein that presents bioactivity of Mycobacterium inducing Th1-type immune reaction without depending upon genetic background and promotes production of interferon γ (INF-γ) inducing activated macrophages important for tuberculosis prevention and differentiation of T cells into CTL.
The vector (rhPIV2-Ag85B) into which foreign genes were introduced as a secretion type was prepared for use as a tuberculosis vaccine, and expression of Ag85B was confirmed. Consequently, as shown in FIG. 5, expression of Ag85B as protein was recognized.
When F expression cells were infected with GFP-PIV2(ΔF), expression and recovery of GFP-PIV2(ΔF) were found to be executable since increasing GFP fluorescence was recognized, as shown in FIG. 6. Furthermore, regarding ΔF/rhPIV2-Ag85B(BCG) vector into which BCG-Ag85B gene was introduced, BCG-Ag85B protein was found to be expressed (FIG. 7).

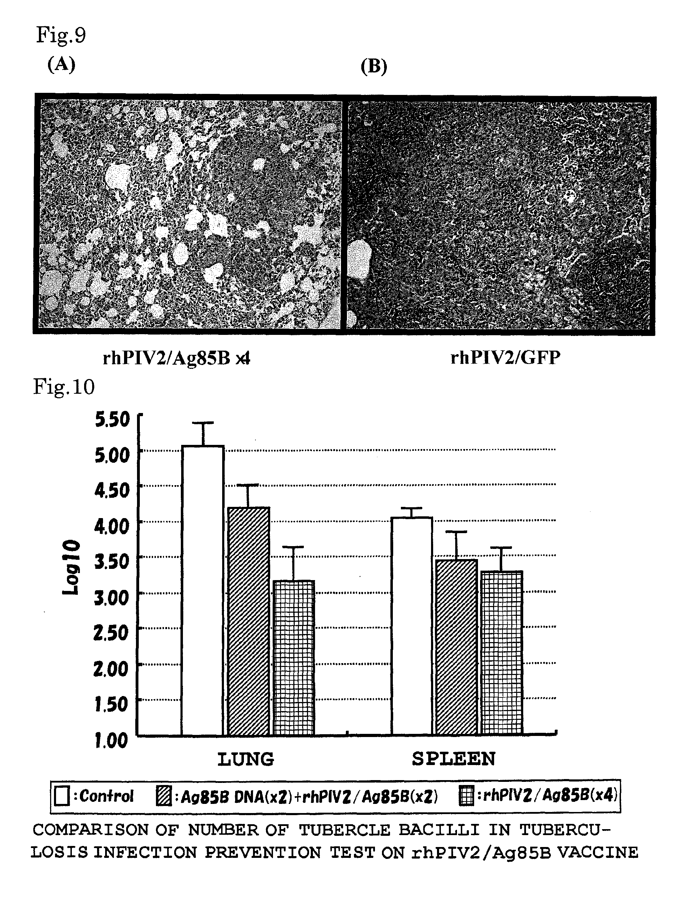

Furthermore, when the mice to which rhPIV2(M-stop), rhPIV2(ΔF) and rhPIV2(ΔM) had been administered were infected with Ag85B or BCG-Ag85B, lesion (tubercle) peculiar to lung tuberculosis due to highly-virulent tubercle bacillus (Mycobacterium tuberculosis Kurono strain) in the lungs was found to decrease significantly. Of these, FIGS. 8 to 10 show the results in the case of rhPIV2-Ag85B. Although FIG. 8(A) shows many tubercles, FIGS. 8(B) and 8(C) show that the number of tubercles was significantly reduced as compared with the control group in turn. FIG. 9 shows photomicrographs of tubercles. Substantially the same state as the normal state is confirmed regarding test group 2 (A) although alveoli of the lung are broken regarding control group (B). Regarding rhPIV2(ΔF) and rhPIV2(ΔM), the similar effect to the foregoing is recognized in the infection with Ag85B and BCG-Ag85B.
FIG. 10 shows the numbers of tubercle bacilli in the lung and the spleen. Regarding control group, 10^{5.05} bacilli were counted in the lung and 10^{4.08} bacilli were counted in the spleen. On the other hand, regarding test group 1, 10^{4.20} bacilli were counted in the lung and 10^{3.45} bacilli were counted in the spleen. Regarding test group 2, 10^{3.10} bacilli were counted in the lung and 10^{3.28} bacilli were counted in the spleen. Generally, it is difficult to permit meaningful differences in the counting of bacilli in conventional vaccine tests except for BCG. The difference of ten times as large as in the number of bacilli is uncommon between the test groups and the control group. In the present test, however, the number of tubercle bacilli in the test group using rhPIV2-Ag85B is about 1/10 to 1/100 of the number of tubercle bacilli in the control group, whereby the vaccine of the embodiment has an exceedingly high preventive effect.

### <Consideration of tuberculosis vaccine of the embodiment>

The hPIV2 prototype vector is specifically infected with an airway mucosa and has a negative nonsegmented single-stranded RNA as a genome. The RNA genome comprises hexanucleotide surrounded by a single nucleocapsid protein to be constructed into a helical ribonucleocapsidprotein (RNP). Since the hPIV2 prototype vector has the nonsegmented RNA as the genome, the hPIV2 prototype vector does not cause any intersegmental variation (discontinuous variation). Furthermore, the effects of transcription and replication are dramatically reduced when the nucleotide number of genomes is not any multiple of 6, whereupon gene mutation due to homologous recombination is highly unlikely. A risk such as accidental homologous recombination is unavoidable, since the above-mentioned MVA85A (vaccinia Ankara) and Aeras402 (adenovirus type 35) both of which are currently in the clinical test phase originate from DNA viruses respectively. In the embodiment, however, the hPIV2 vector originates from the RNA virus and the life cycle is completed within cell cytoplasm. Accordingly, differing from the above-mentioned two DNA vectors, the hPIV2 vector can prevent genome from being integrated into chromosome in the nucleus.

The vector from which expression of M, F and HN genes is eliminated by stop codon or genetic deletion is an RNP machine that has no risk of production of secondary infection particle and can intranasally introduce genes specifically into airway mucosa. The vector has a lower original pathogenicity than the above-mentioned DNA vectors, and it has not been reported that the vector is pathogenic to humans (adults). Thus, the vector has an exceeding safety. Furthermore, the vector is designed so that expression of foreign genes from the genome becomes maximum by maximizing the characteristic of transcription from the hPIV2 genome. Furthermore, the M, F or HN gene is considered to suppress transcription from the genome. Accordingly, the protein expression level of the vector from which products of M, F and HN genes have been deleted is exceedingly high. A high intensity of expression of GFP was confirmed when the rhPIV2 (M stop)-GFP into which GFP (green fluorescent protein) had been inserted was intranasally administered to hamsters (FIG. 4).

Importance is given to induction of local immunity (cell mediated immunity) in the lung that is a disease expression site than to general immunity in tuberculosis prevention. In the embodiment, the nongrowing rhPIV2-Ag85B vector or rhPIV2-BCG-Ag85B vector into each of which cell-mediated immunity induction protein had been inserted was used as a vaccine. Since this vector is safe and can realize an intense expression of the cell-mediated immunity induction protein to the lung, the vector was considered to have an exceeding efficacy as an adult tuberculosis vaccine vector.
This vaccine vector was developed as a safe half-live attenuated intranasal spray-type tuberculosis vaccine. The α antigen (Ag85B) derived from atypical mycobacterium (Mycobacterium kansasii and Mycobacterium bovisBCG) encoded as a foreign antigen has a cell-mediated immunity inducing function. A high cell-mediated immunity (Th1) against tubercle bacilli was considered to be induced by highly expressing Ag85B in a lung that was a disease expression section. Actually, when hamsters were nasally infected with an rhPIV2 vector, intense expression of GFP which was a marker gene was found in airway epithelial cell and even in terminal bronchial (FIG. 4).

The test for protecting against infection with use of mice (the tuberculosis prevention test) confirmed that rhPIV2-Ag85 had an intense tuberculosis preventing efficacy (FIGS. 8 to 10). In this test protocol, the efficacy of combined administration of expression type Ag85B-DNA and rhPIV2-Ag85B (test group 1) was examined in addition to administration of rhPIV2-Ag85B alone. An exceedingly high preventive efficacy was confirmed in test group 2, whereas only limited efficacy was confirmed in test group 1. Conventional reports indicate that a preventive efficacy was also confirmed regarding expression type Ag85B-DNA. In this time's test results, however, the preventive efficacy of expression type Ag85B-DNA is considered to be limited, and large part of preventive efficacy of test group 1 is considered to depend upon rhPIV2-Ag85B.
Furthermore, the similar preventive efficacy to rhPIV2-Ag85B was achieved from hgPIV2-Ag85B to which BCG-Ag85B was introduced instead of Ag85B.
Furthermore, there is a high possibility that each of Ag85A and Ag85c achieves the similar efficacy from the test similar to the foregoing test.

According to the above-described embodiment, it is clear that a noticeable efficacy in prevention of tuberculosis can be achieved by administering the expression vector in which the gene encoding the α antigen or the like has been integrated in the paramyxovirus (particularly, rhPIV2).

## Claims

1. A intranasal spray-type tuberculosis vaccine, comprising a gene encoding an α antigen derived from Mycobacteria, an analog thereof or a mutant thereof having a function similar thereto, said gene being integrated into a paramyxovirus gene deficient in an M gene, F gene or HN gene.

2. The intranasal spray-type tuberculosis vaccine according to claim 1, wherein the α antigen is derived from Mycobacterium kansasii or Mycobacterium bovis BCG.

3. The intranasal spray-type tuberculosis vaccine according to claim 1 or 2, wherein the analog of the α antigen is an 85 complex-forming protein 85A or 85 complex-forming protein 85C.

4. A method of preventing infection of tuberculosis, comprising:
integrating a gene encoding an α antigen derived from Mycobacteria, an analog thereof or a mutant having a function similar thereto into a paramyxovirus gene deficient in an M gene, F gene or HN gene; and
nebulizing a human for intranasal administration.

5. The method according to claim 4, wherein the α antigen is derived from Mycobacterium kansasii or Mycrobacterium bovis BCG.

6. The method according to claim 4 or 5, wherein the analog of the α antigen is an 85 complex-forming protein 85A or 85 complex-forming protein 85C.
